# EUROPEAN PATENT SPECIFICATION

(11) **EP 3 128 327 B1**
(45) Date of publication and mention of the grant of the patent: **12.12.2018**
(21) Application number: 15773282.7
(22) Date of filing: 03.04.2015
(51) Int. Cl.: G01N 33/68, G01N 21/64, G01N 33/48, G01N 33/49, C07K 14/435

(54) **METHOD FOR DETECTING SMN PROTEIN EXPRESSION**
VERFAHREN ZUR DETEKTION EINER SMN-PROTEIN-EXPRESSION
PROCÉDÉ DE DÉTECTION DE L'EXPRESSION DE LA PROTÉINE SMN

(30) Priority: 03.04.2014 JP 2014076985
(43) Date of publication of application: 08.02.2017
(73) Proprietor: Tokyo Women's Medical University, Tokyo 162-8666 (JP); Microbial Chemistry Research Foundation, Tokyo 141-0021 (JP)
(72) Inventor: SAITO, Kayoko, Tokyo 162-8666 (JP); ARAKAWA, Reiko, Tokyo 162-8666 (JP); ARAKAWA, Masayuki, Tokyo 141-0021 (JP); NOMOTO, Akio, Tokyo 141-0021 (JP)
(74) Representative: Marro, Nicolas
(86) International application number: PCT/JP2015/060662
(87) International publication number: WO 2015/152410

(56) References cited:
- JP-A- H06 501 106
- DIONE T. KOBAYASHI ET AL: "Evaluation of Peripheral Blood Mononuclear Cell Processing and Analysis for Survival Motor Neuron Protein", PLOS ONE, vol. 7, no. 11, 30 November 2012 (2012-11-30), page e50763, XP055389287, DOI: 10.1371/journal.pone.0050763
- KOLB STEPHEN J ET AL: "A novel cell immunoassay to measure survival of motor neurons protein in blood cells", BMC NEUROLOGY, BIOMED CENTRAL LTD., LONDON, GB, vol. 6, no. 1, 1 February 2006 (2006-02-01), page 6, XP021015952, ISSN: 1471-2377, DOI: 10.1186/1471-2377-6-6
- PHILIP J. YOUNG ET AL: "The Relationship between SMN, the Spinal Muscular Atrophy Protein, and Nuclear Coiled Bodies in Differentiated Tissues and Cultured Cells", EXPERIMENTAL CELL RESEARCH, vol. 256, no. 2, 1 May 2000 (2000-05-01), pages 365-374, XP055389288, AMSTERDAM, NL ISSN: 0014-4827, DOI: 10.1006/excr.2000.4858
- ARAKAWA MASAYUKI ET AL: "A novel evaluation method of survival motor neuron protein as a biomarker of spinal muscular atrophy by imaging flow cytometry", BIOCHEMICAL AND BIOPHYSICAL RESEARCH COMMUNICATIONS, ELSEVIER, AMSTERDAM, NL, vol. 453, no. 3, 27 September 2014 (2014-09-27), pages 368-374, XP029117054, ISSN: 0006-291X, DOI: 10.1016/J.BBRC.2014.09.087
- ARAKAWA REIKO ET AL: "Imaging Flow Cytometry Analysis to Identify Differences of Survival Motor Neuron Protein Expression in Patients With Spinal Muscular Atrophy", PEDIATRIC NEUROLOGY, ELSEVIER SCIENCE, NL, vol. 61, 26 May 2016 (2016-05-26), pages 70-75, XP029679321, ISSN: 0887-8994, DOI: 10.1016/J.PEDIATRNEUROL.2016.05.009
- AKIO NOMOTO: 'Biomarker to shite no SMN Tanpakushitsu Sokuteiho ni Kansuru Kenkyu' SHONIKI HASSHO SEKIZUISEI KIN'ISHUKUSHO NI TAISURU SODIUM VALPROATE TASHISETSU KYODO ISHI SHUDO CHIKEN JUNBI KENKYU HEISEI 25 NENDO SOKATSU-BUNTAN KENKYU HOKOKUSHO March 2014, pages 23 - 24, XP008185732
- JONATHAN J. CHERRY: 'Enhancement of SMN protein levels in a mouse model of spinal muscular atrophy using novel drug-like compounds' EMBO MOLECULAR MEDICINE vol. 5, 14 July 2013, pages 1103 - 1118, XP055359199 'Increases in total SMN protein levels and SMN gems in SMA derived fibroblasts' MATERIALS AND METHODS pages 1114 - 1115
- Copy of the journal article by Akio Nomoto cited on the International Search Report and accompanying attachments regarding date of publication.

## Description

### Technical Field

The present invention relates to a method for detecting the expression of survival motor neuron (SMN) protein.

### Background Art

Spinal muscular atrophy (SMA) is a muscular atrophy caused by a lesion of anterior horn cells of the spinal cord, and shows a lower motor neuron sign characterized by muscle weakness and muscle atrophy in the trunk and limbs. SMA is classified into type I to type IV according to the age of onset and severity: type I which is severe type (also referred to as Werdnig-Hoffmann disease) and occurs by the age of six months after birth; type II which is intermediate type (also referred to as Dubowitz disease) and occurs by the age of one year and six months; and type III which is mild type (also referred to as Kugelberg-Welander disease) and occurs after the age of one year and six months. These are childhood SMA. On the other hand, type IV which occurs at the age of 20 years or older is adult SMA.

SMA type I accounts for approximately 30% of SMA and occurs at the age of six months or younger. The symptom is quite serious. The affected individual cannot keep sitting throughout life, and can rarely survive for two years or longer without artificial respiration. SMA type II makes it impossible for the affected individual to stand up and walk throughout life. The person affected with SMA type III can walk independently, but a symptom gradually appears such that the person tends to fall over, cannot walk, or cannot stand up. Despite these facts, the method for completely curing SMA has not been established yet, and SMA is one of diseases designated as intractable diseases by the country.

The responsible gene in many childhood SMA cases is the SMN1 gene located in 5q13 on a long arm of chromosome 5. In many childhood SMA cases, a deletion or mutation of the SMN1 gene has been observed, so that childhood SMA is recognized as an autosomal recessive genetic disorder. The SMN1 gene expresses SMN protein, and the SMN protein is conceivably involved in the development and maintenance of spinal motor neuron and so forth.

In addition, in the same region on chromosome 5 where the SMN1 gene is located, the SMN2 gene exists which is different from the SMN1 gene by only one base in the coding region. In childhood SMA patients, the SMN1 gene is deleted or mutated, the SMN protein is decreased because only SMN2 gene functions. It is believed that the severity of the symptom varies depending on the amount of the SMN2-gene derived SMN protein expressed.

However, while the transcription product of the SMN1 gene is full-length SMN mRNA, assuming that the amount of full-length SMN mRNA transcribed from the SMN1 gene is 100%, full-length SMN mRNA of the SMN2 gene accounts for approximately 10% of the transcription product thereof, and truncated SMN mRNA in which deletion of exon 7 is observed accounts for approximately 90%. The truncated SMN mRNA is translated into a non-functional protein, and only the full-length SMN mRNA is normally translated into the SMN protein. Therefore, in childhood SMA patients in which the SMN1 gene is deleted or mutated, the amount of the SMN protein expressed is low in comparison with healthy persons and is only about 10% to 20% of that in healthy persons. Hence, the deletion or mutation of the SMN1 gene causes muscle weakness and muscle atrophy.

In childhood SMA patients having the SMN1 gene deleted, the SMN2 gene may be present in place of the SMN1 gene. In this case, two copies of the SMN2 gene exist on one chromosome. The amount of the SMN2-gene derived SMN protein expressed is also increased depending on the number of copies. It has been known that, in childhood SMA patients, the larger the amount of the SMN2-gene derived SMN protein expressed, the milder the symptom.

As described above, the amount of the SMN protein expressed is closely related to childhood SMA. It has been believed that the SMN protein can be a biomarker useful for diagnosing childhood SMA and for accurately determining the effect of an agent. However, in childhood SMA patients also, the expression of the SMN protein per se is observed, and utilizing the SMN protein as a biomarker useful for childhood SMA requires some sensitivity enough to detect a difference in the level of the SMN protein expressed when a patient is compared with a healthy person.

Moreover, childhood SMA is an autosomal recessive genetic disorder, and when the SMN1 gene is deleted or mutated in one of a pair of autosomes, the symptoms of muscle weakness and muscle atrophy do not appear at all, but the person is a carrier. In the carrier, the amount of the SMN protein expressed is larger than that in the patient. However, the difference between a carrier and a patient is presumably not always as large as the difference between a healthy person and a patient in the amount of the SMN protein expressed. Thus, utilizing the SMN protein as a biomarker useful for childhood SMA requires further sensitivity enough to detect a difference in the level of the SMN protein expressed when a carrier is compared with a patient.

A quantitative analysis has been actually attempted on the SMN protein by an ELISA method using peripheral blood mononuclear cells (Dione T. Kobayashi et al., Plos one, November 2012, Vol. 7, Issue 11, e50763, Evaluation of Peripheral Blood Mononuclear Cell Processing and Analysis for Survival Motor Neuron Protein) . However, Kobayashi et al. have reported that the result obtained by the ELISA method showed no significant difference in the level of the SMN protein expressed between childhood SMA patients and carriers. Further, Kobayashi et al. have even disclosed that the level of the SMN protein expressed does not serve as a reliable indicator in the SMA diagnosis.

Moreover, in the SMN protein quantitative analysis by the ELISA method, peripheral blood mononuclear cells (PBMCs) have to be used, and operations such as centrifuging blood samples obtained from patients and others are burdensome, and also reduce the cell yield. Hence, the SMN protein quantification by the ELISA method using PBMCs also requires a large amount of blood to be collected.

As has been described above, the conventional techniques have various problems in utilizing the SMN protein as a biomarker useful for childhood SMA. These have presented major obstacles to the establishment of the method for completely curing SMA.

In addition, there has been no report so far on reliable SMN protein detection method capable of detecting a difference in the level of the SMN protein expressed when a childhood SMA patient is compared with a healthy person by using blood samples such as whole blood (peripheral blood) or samples obtained by hemolyzing red blood cells in whole blood.

Heretofore, no reliable method for detecting SMN protein expression by using blood cell samples, which is capable of detecting a difference in the level of the SMN protein expressed between a childhood SMA patient and a healthy person and/or between a childhood SMA patient and a carrier thereof, has been found.

An object of the present invention is to provide a reliable method for detecting SMN protein expression by using blood cell samples, the method being capable of detecting a difference in the level of the SMN protein expressed between a childhood SMA patient and a healthy person and/or between a childhood SMA patient and a carrier thereof.

The present inventors have conducted earnest studies and as a result, the inventors have newly found out that the sensitivity of detecting the level of the SMN protein expressed is enhanced, by selecting a particular cell population among blood cells in which SMN protein expression is to be detected and detecting the SMN protein expression in the cell population.

### Summary of Invention

One aspect of the present invention provides a method for detecting the expression of SMN protein, comprising the steps of:
labeling SMN protein in a sample containing nucleated cells derived from blood with a first fluorescent dye;
labeling the nuclei of the nucleated cells in the sample;
selecting a cell population of the nucleated cells in which nuclei and SMN protein are labeled; and
detecting the expression of the SMN protein based on the label for the SMN proteins in the selected cell population, wherein said detection step comprises measuring an amount of the SMN protein expressed.

According to one aspect of the present invention, in the method for detecting SMN protein expression, the step of detecting the expression of the SMN protein comprises measuring a fluorescence intensity emitted by the first fluorescent dye.

According to one aspect of the present invention, in the method for detecting SMN protein expression, the step of selecting a cell population of the nucleated cells in which nuclei and SMN protein are labeled with a fluorescent dye comprises measuring, in a single device, a first fluorescence intensity emitted by the first fluorescent dye and a second fluorescence intensity emitted by the second fluorescent dye, and representing the measured first and second fluorescence intensities in two-dimensional coordinates.

According to one aspect of the present invention, in the method for detecting SMN protein expression, the step of selecting a cell population of the nucleated cells in which nuclei and SMN protein are labeled with a fluorescent dye and the step of detecting the expression of the SMN protein, wherein said detection step comprises measuring an amount of the SMN protein expressed, are performed by imaging flow cytometry.

According to one aspect of the present invention, in the method for detecting SMN protein expression, the sample containing the nucleated cells derived from the blood includes nucleated cells obtained by hemolyzing red blood cells in the blood and then centrifuging the resultant to precipitate the nucleated cells.

One aspect of the present invention provides a method for detecting SMN protein, comprising the steps of:
centrifuging blood to prepare the sample containing nucleated cells derived from the blood before the step of labeling SMN protein in the sample containing the nucleated cells derived from the blood.

According to one aspect of the present disclosure the blood is obtained from a test subject, the step of detecting the expression of the SMN protein comprises measuring an amount of the SMN protein expressed, the method comprises a step of comparing the amount of the SMN protein expressed with a control selected from the following (a) to (c):
(a) if the test subject is a childhood spinal muscular EP 15 773 282.7 3 / 3 atrophy (SMA) patient, the control is an amount of SMN protein expressed measured in the same manner as the process for measuring the expressed amount of the SMN protein by using the blood obtained from the test subject, except that blood obtained from a healthy person or a carrier is used;
(b) if the test subject is a healthy person or a carrier, the control is an amount of SMN protein expressed measured in the same manner as the process for measuring the expressed amount of the SMN protein by using the blood obtained from the test subject, except that blood obtained from a childhood SMA patient is used; or
(c) if the test subject is a person to which a drug or a drug candidate against SMA has been administered, the control is an amount of SMN protein expressed measured in the same manner as the process for measuring the expressed amount of the SMN protein by using the blood obtained from the test subject after the administration of the drug or the drug candidate against SMA, except that blood obtained from the test subject before the administration is used.

### Brief Description of Drawings

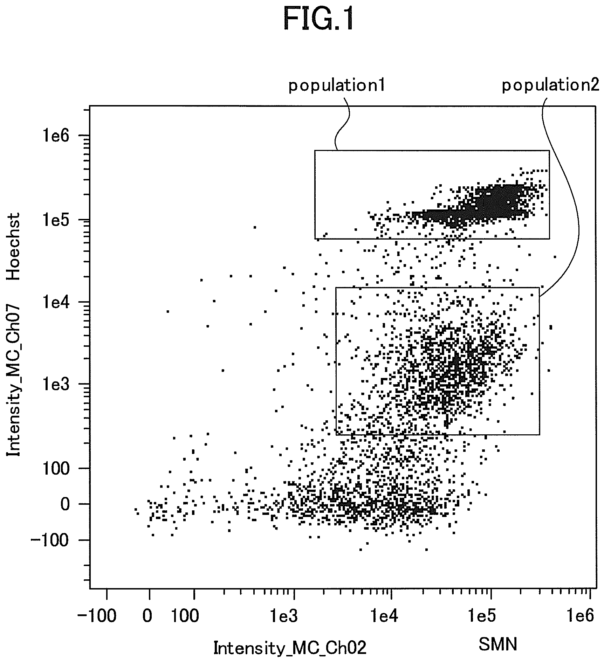

Fig. 1 shows a graph plotting fluorescence intensities measured by imaging flow cytometry using lymphoblasts derived from peripheral blood mononuclear cells obtained from a healthy person. The vertical axis represents a fluorescence intensity emitted by a nuclear staining agent, and the horizontal axis represents a fluorescence intensity emitted by a SMN protein labeling reagent.

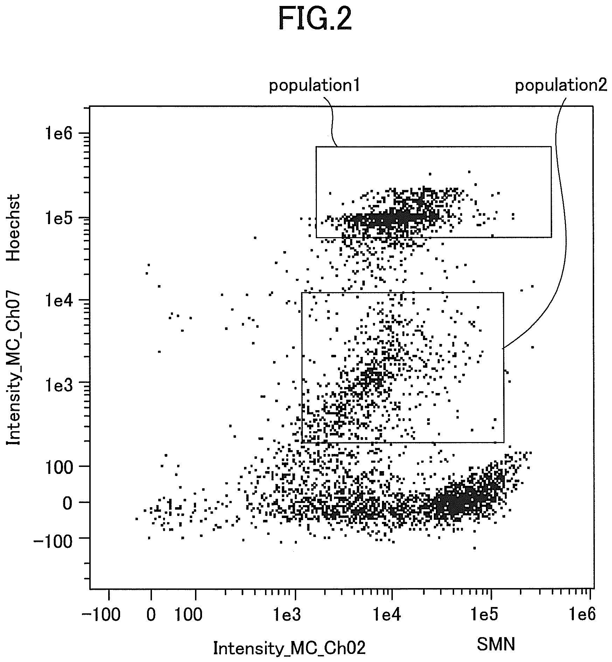

Fig. 2 shows a graph plotting fluorescence intensities measured by imaging flow cytometry using lymphoblasts derived from peripheral blood mononuclear cells obtained from a SMA type I patient. The vertical axis represents a fluorescence intensity emitted by a nuclear staining agent, and the horizontal axis represents a fluorescence intensity emitted by a SMN protein labeling reagent.

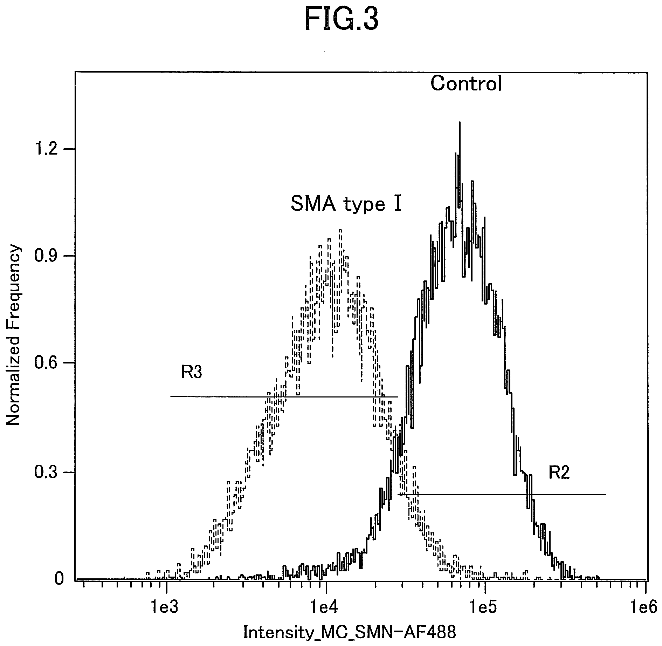

Fig. 3 shows a graph for illustrating a comparison between the fluorescence intensity emitted by the SMN protein labeling reagent measured by the imaging flow cytometry using the lymphoblasts derived from the peripheral blood mononuclear cells obtained from the healthy person with the fluorescence intensity emitted by the SMN protein labeling reagent measured by the imaging flow cytometry using the lymphoblasts derived from the peripheral blood mononuclear cells obtained from the SMA type I patient. The vertical axis represents a relative frequency, and the horizontal axis represents a fluorescence intensity emitted by the SMN protein labeling reagent.

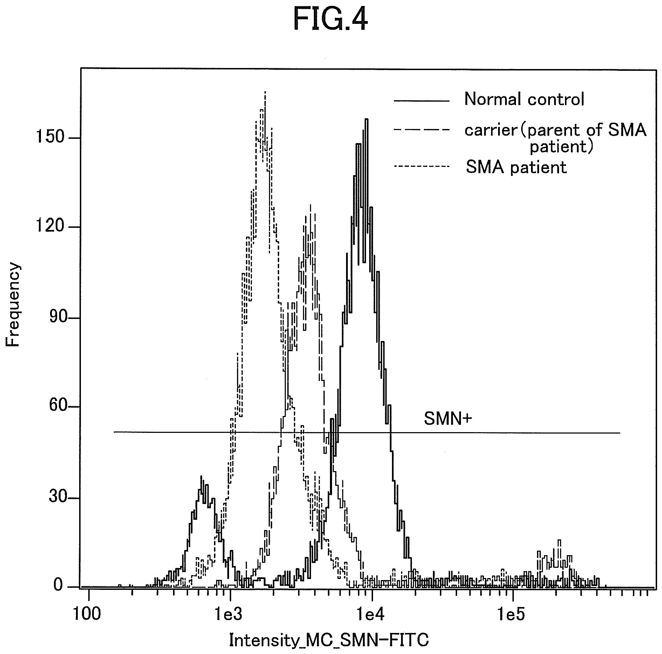

Fig. 4 shows a graph for illustrating a comparison among fluorescence intensities emitted by the SMN protein labeling reagent measured by the imaging flow cytometry using samples obtained by hemolyzing red blood cells in blood obtained respectively from a healthy person, a carrier, and a SMA type I patient. The vertical axis represents a frequency, and the horizontal axis represents a fluorescence intensity emitted by the SMN protein labeling reagent.

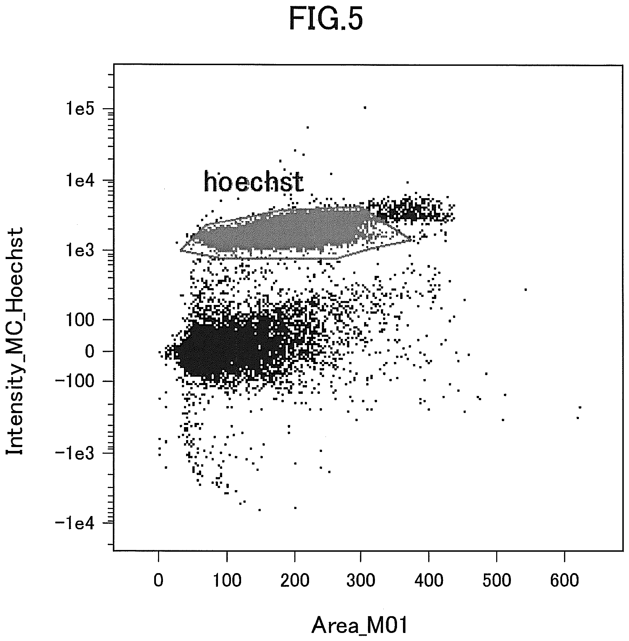

Fig. 5 shows a graph plotting fluorescence intensities measured by the imaging flow cytometry using samples obtained by hemolyzing red blood cells in blood obtained from a healthy person. The vertical axis represents a fluorescence intensity emitted by the nuclear staining agent, and the horizontal axis represents a surface area. In this experiment shown in Fig. 5, 84203 cells were used for the imaging flow cytometry, and 73842 cells were selected as cells in which the nuclei were stained and which kept the surface area (in the graph, indicated by hoechst).

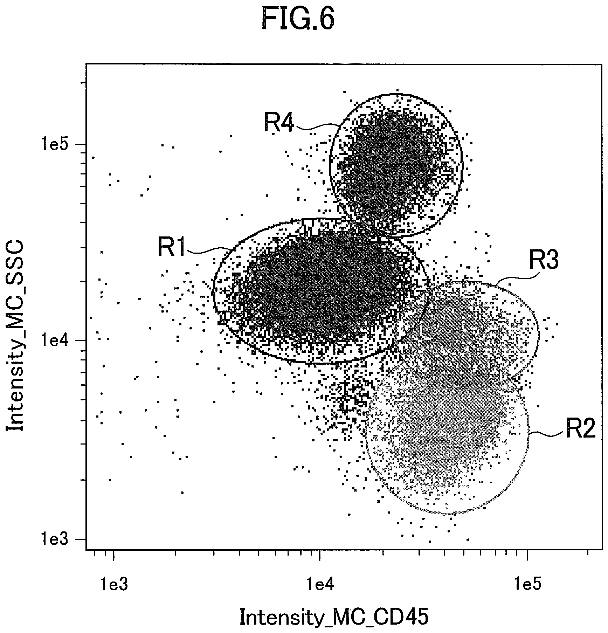

Fig. 6 shows a graph plotting fluorescence intensities measured by the imaging flow cytometry using the samples obtained by hemolyzing the red blood cells in the blood obtained from the healthy person. The vertical axis represents side scatter (SSC), and the horizontal axis represents a fluorescence intensity emitted by a fluorescent dye conjugated to an anti-CD45 antibody.

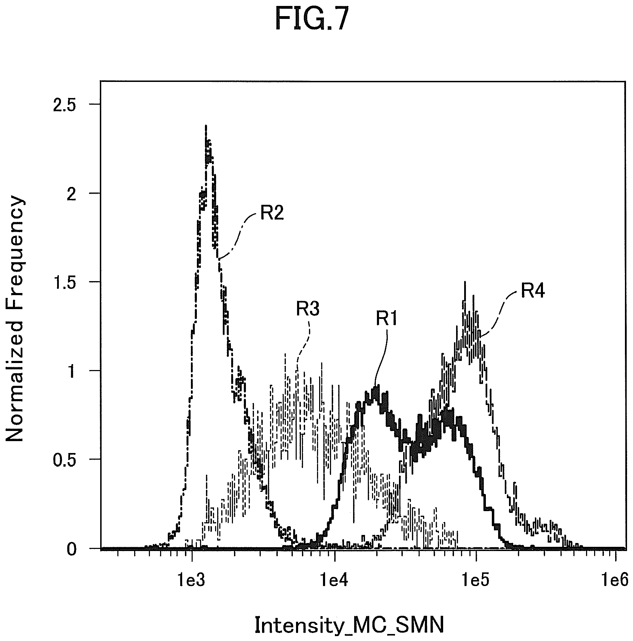

Fig. 7 shows a graph for illustrating a comparison among fluorescence intensities emitted by the SMN protein labeling reagent in clusters, measured by the imaging flow cytometry using the samples obtained by hemolyzing the red blood cells in the blood obtained from the healthy person. The vertical axis represents a relative frequency, and the horizontal axis represents a fluorescence intensity emitted by the SMN protein labeling reagent.

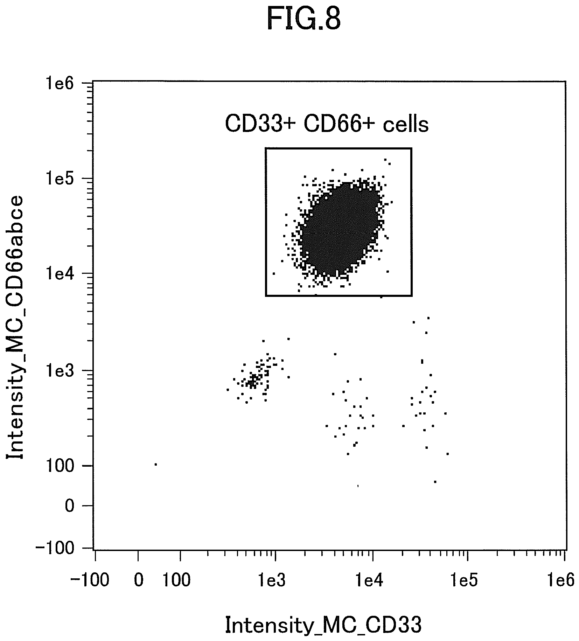

Fig. 8 shows a graph plotting fluorescence intensities measured by the imaging flow cytometry using the samples obtained by hemolyzing the red blood cells in the blood obtained from the healthy person. The vertical axis represents a fluorescence intensity emitted by a fluorescent dye conjugated to an anti-CD66abce antibody, and the horizontal axis represents a fluorescence intensity emitted by a fluorescent dye conjugated to an anti-CD33 antibody.

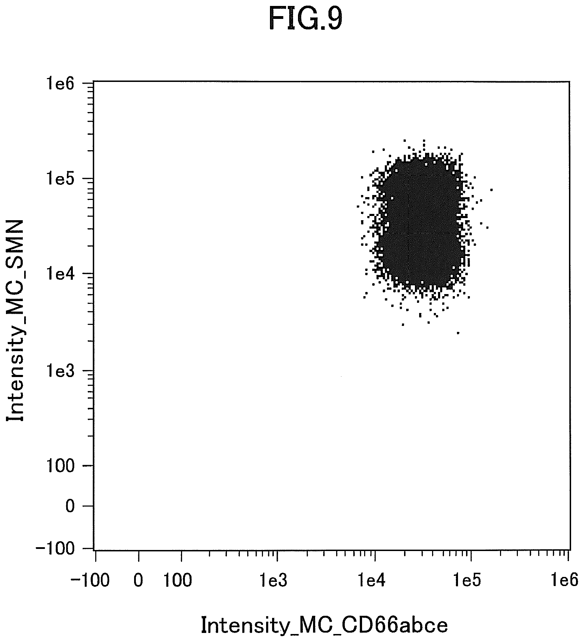

Fig. 9 shows a graph plotting fluorescence intensities measured by the imaging flow cytometry using the samples obtained by hemolyzing the red blood cells in the blood obtained from the healthy person. The vertical axis represents a fluorescence intensity emitted by the SMN protein labeling reagent, and the horizontal axis represents a fluorescence intensity emitted by a fluorescent dye conjugated to an anti-CD66abce antibody.

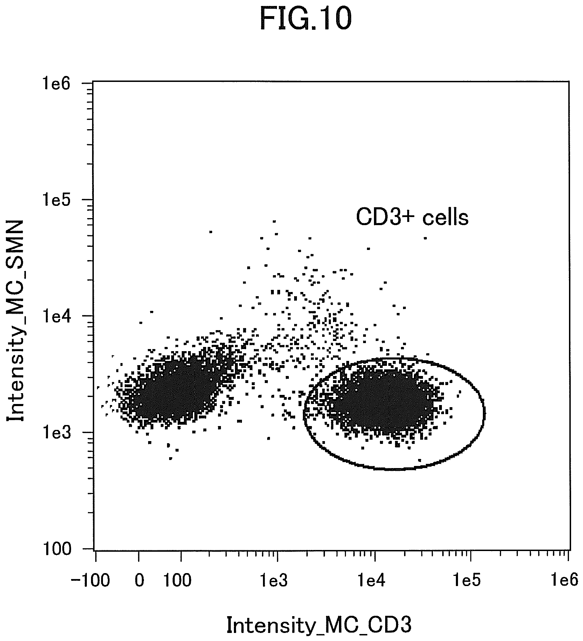

Fig. 10 shows a graph plotting fluorescence intensities measured by the imaging flow cytometry using the samples obtained by hemolyzing the red blood cells in the blood obtained from the healthy person. The vertical axis represents a fluorescence intensity emitted by the SMN protein labeling reagent, and the horizontal axis represents a fluorescence intensity emitted by a fluorescent dye conjugated to an anti-CD3 antibody.

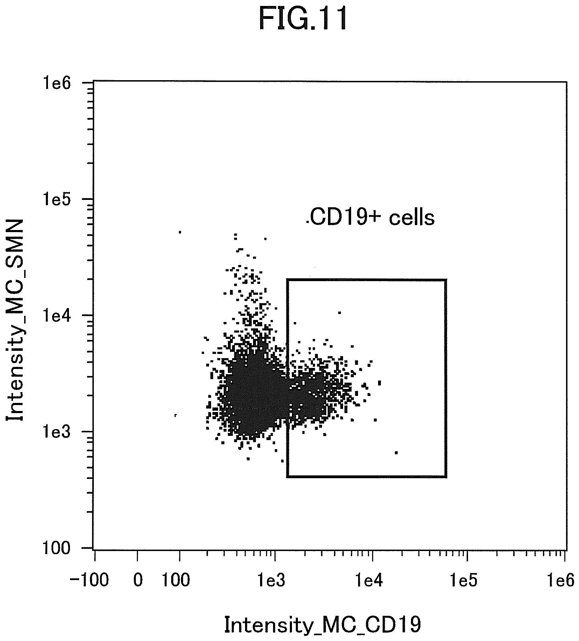

Fig. 11 shows a graph plotting fluorescence intensities measured by the imaging flow cytometry using the samples obtained by hemolyzing the red blood cells in the blood obtained from the healthy person. The vertical axis represents a fluorescence intensity emitted by the SMN protein labeling reagent, and the horizontal axis represents a fluorescence intensity emitted by a fluorescent dye conjugated to an anti-CD19 antibody.

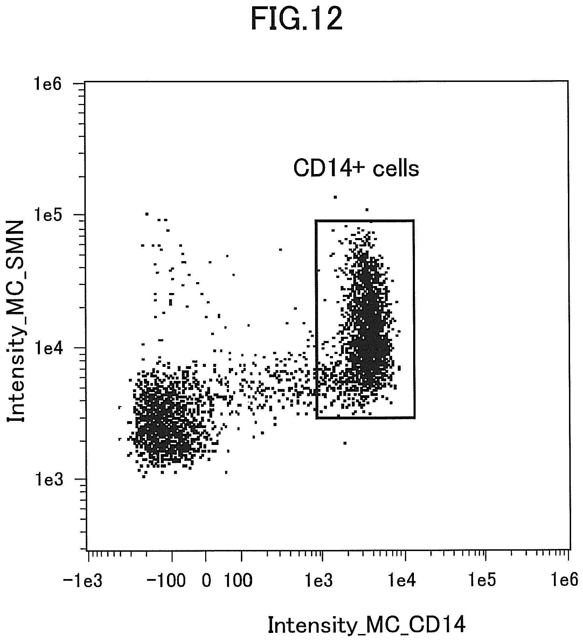

Fig. 12 shows a graph plotting fluorescence intensities measured by the imaging flow cytometry using the samples obtained by hemolyzing the red blood cells in the blood obtained from the healthy person. The vertical axis represents a fluorescence intensity emitted by the SMN protein labeling reagent, and the horizontal axis represents a fluorescence intensity emitted by a fluorescent dye conjugated to an anti-CD14 antibody.

### Description of Embodiments

Hereinafter, the present invention will be described in detail.

In a method for detecting SMN protein expression of the present invention, a sample derived from blood obtained from a test subject is used. Such a blood sample is less invasive to a patient and best suited as a specimen. The subject is, for example, a childhood SMA patient, a carrier thereof, or a person who is neither a childhood SMA patient nor a carrier thereof (healthy person), and is not particularly limited. The childhood SMA patient includes a SMA type I patient, a SMA type II patient, and a SMA type III patient.

The sample derived from blood obtained from a test subject, which is used in the method of the present invention, should contain nucleated cells derived from the blood. As long as the sample derived from blood obtained from a test subject, which is used in the method of the present invention, is treated in such a manner that the sample contains nucleated cells derived from the blood, the blood obtained from test subject may be treated in any manner. For example, as the sample containing nucleated cells derived from blood used in the method of the present invention, it is possible to use, but is not limited to, peripheral blood mononuclear cells (PBMCs) separated by centrifuging blood obtained from a test subject; and a sample containing nucleated cells obtained by hemolyzing red blood cells in blood and then centrifuging the resultant to precipitate it.

In the method of the present invention, techniques conventionally known in the technical field of the present invention can be used to treat blood obtained from a test subject so as to obtain a sample containing nucleated cells derived from the blood.

In the method of the present invention, the method for treating blood obtained from a test subject to obtain a sample containing nucleated cells derived from the blood is preferably a method which results in a favorable cell yield, and by which cells are damaged as little as possible. For example, a method comprising hemolyzing red blood cells in blood, and then separating nucleated cells precipitated by centrifugation (lysis method) requires only a small amount of blood to be collected from a test subject, and also imposes only a small stress to the blood cells. For these reasons, in the method of the present invention, the lysis method is preferably used to treat blood obtained from a test subject so as to obtain a sample containing nucleated cells derived from the blood. Particularly, when SMN protein is utilized as a biomarker in testing a drug or a drug candidate against SMA, the lysis method is advantageously used because it requires only a small amount of blood to be collected from a child patient or the like.

Moreover, in the method of the present invention, as the sample containing nucleated cells derived from blood, a sample containing lymphoblasts transformed from nucleated cells derived from blood after the cells are separated by conventionally known techniques as described above may be used. For the lymphoblast transformation treatment also, techniques conventionally known in the technical field of the present invention can be used.

One embodiment of the method of the present invention comprises a step of centrifuging blood to prepare a sample containing nucleated cells derived from the blood. The blood centrifugation can be performed, for example, at approximately 1400 to approximately 3200 rpm for approximately 5 to approximately 20 minutes.

In a case where the step of centrifuging blood to prepare a sample containing nucleated cells derived from the blood comprises separating the nucleated cells by the lysis method, the hemolysis with a lysing agent is followed by the centrifugation which is performed preferably at approximately 1400 to approximately 2300 rpm for approximately 5 to approximately 8 minutes.

In a case where the step of centrifuging blood to prepare a sample containing nucleated cells derived from the blood comprises separating PBMCs, it is preferable to collect the blood into a heparin tube and have the blood overlaid on a Ficoll solution, or to place the blood in BD Vacutainer(registered trademark) CPT™, which is a blood-collecting tube for separating mononuclear cells, followed by the centrifugation which is performed preferably at approximately 2000 to approximately 3200 rpm for approximately 15 to approximately 20 minutes.

The step of centrifuging blood to prepare a sample containing nucleated cells derived from the blood can be performed using techniques conventionally known in the technical field of the present invention.

The amount of the blood derived from a test subject, which is used in the method of the present invention, is not particularly limited as long as the SMN protein can be expressed. For example, the amount is approximately 0.5 mL or more, preferably approximately 1 mL or more, and approximately 3 mL or less, preferably approximately 2 mL or less. Note that, in the conventional quantitative analysis on the SMN protein by the ELISA method using peripheral blood mononuclear cells (PBMCs), the amount of blood required is at least 4 mL. Thus, one embodiment of the present invention is advantageous in that the amount of blood required is small.

The method for detecting SMN protein expression of the present invention comprises a step of labeling SMN protein in a sample containing nucleated cells derived from blood with a first fluorescent dye.

The SMN protein is a protein expressed by SMN gene which is a SMA responsible gene, and conceivably involved in motor neuron formation and so forth. The SMN gene includes the SMN1 gene and the SMN2 gene. Both are located in 5q13 on a long arm of chromosome 5. The SMN2 gene is different from the SMN1 gene by only one base in the coding region. However, assuming that the amount of full-length SMN mRNA transcribed from the SMN1 gene and translated into a normal SMN protein is 100%, full-length SMN mRNA of the SMN2 gene accounts for approximately 10% of the transcription product, and truncated SMN mRNA in which deletion of exon 7 is observed accounts for approximately 90%. The truncated SMN mRNA produces a non-functional protein, and has no use for the development and maintenance of spinal motor neuron.

In the method of the present invention, techniques conventionally known in the technical field of the present invention can be used to label SMN protein with a first fluorescent dye in a sample containing nucleated cells derived from blood. Herein, labeling the SMN protein with a first fluorescent dye should be able to label a normal SMN protein translated from full-length SMN mRNA, but may also label a non-functional protein translated from the truncated SMN mRNA.

Examples of the method for labeling the SMN protein used in the method of the present invention include, but is not limited to, the use of an anti-SMN antibody conjugated to a labeling reagent, wherein said labeling reagent is a fluorescent dye; and the use of an anti-SMN antibody as a primary antibody not conjugated to a labeling reagent followed by the use of a secondary antibody conjugated to a labeling reagent, wherein said labeling reagent is a fluorescent dye. Examples of the labeling reagent include enzymes, dyes, fluorescent dyes, biotin, radioactive substances, various peptides, and amino acid linkers, but are not limited thereto. According to the invention, the labeling reagent is a fluorescent dye, and examples thereof include fluorescein, rhodamine, coumarin, imidazole derivatives, indole derivatives, Cy3, Cy5, Cy5.5, Cy7, APC, PE, DyLight, AlexaFluor, and the like.

The anti-SMN antibody conjugated to a labeling reagent, wherein said labeling reagent is a fluorescent dye, or the anti-SMN antibody used as a primary antibody, which is used in the method of the present invention, may be a monoclonal antibody or a polyclonal antibody, and commercially available products can be utilized. Examples of commercially available products of the anti-SMN antibodies include Milli-Mark™ of Millipore Corporation, antibodies provided by BD, Abcam plc., and Sigma-Aldrich Co., and other similar antibodies.

The method for detecting SMN protein expression of the present invention comprises a step of labeling a nuclei of the nucleated cells in the sample containing the nucleated cells derived from blood with a second fluorescent dye. As the method for labeling the nuclei, techniques conventionally known in the technical field of the present invention can be used. Examples of the method for labeling the nuclei include labeling with a fluorescent dye; labeling with an antibody capable of recognizing a cell nucleus-specific protein; and the like.

In the method of the present invention, a fluorescent dye is used to label the nuclei of the nucleated cells in the sample containing the nucleated cells derived from blood, examples of the reagent include Hoechst 33342, Hoechst 33258, 4,6-diamino-2-phenylindole (DAPI), Propidium iodide (PI), fluorescence-labeled anti-histone antibodies, fluorescence-labeled anti-lamin antibodies, and the like.

The method for detecting SMN protein expression of the present invention comprises a step of selecting a cell population of the nucleated cells in which nuclei and SMN protein are labeled. The cell population of the nucleated cells in which nuclei and SMN protein are labeled with a first fluorescent dye is a population of cells in which nuclei and SMN protein are labeled with a first fluorescent dye as described in the present description.

In one embodiment of the method of the present invention, the cell population of the nucleated cells in which nuclei and SMN protein are labeled with a fluorescent dye includes intact cells (viable cells) by 100%, 95% or more, 90% or more, 85% or more, 80% or more, 75% or more, 70% or more, 65% or more, 60% or more, 55% or more, or 50% or more of the population.

In one embodiment of the method of the present invention, the cell population of the nucleated cells in which nuclei and SMN protein are labeled is a population including cells having a nuclear fluorescence intensity of approximately 1×10⁵ or more and a SMN-protein fluorescence intensity of approximately 1×10³ or more when the nuclei and SMN protein are labeled with a fluorescent dye as described later in Example 1 of the present description to detect the fluorescence intensities as described later in Example 1. Such cells are determined as intact cells.

Additionally, cells keeping the cell form and also keeping the aspect ratio (width-to-height ratio) and surface area under microscope observation are determined as intact cells.

The criteria of determining intact cells or determining a cell population including intact cells vary depending on the method for labeling nuclei and SMN protein with a fluorescent dye in nucleated cells. However, the determinations can be made as appropriate based on the results of detecting nuclei and SMN protein in nucleated cells on the basis of a labeling reagent to be used.

In the method of the present invention, the nucleated cells to be detected preferably include lymphocytes, monocytes, granulocytes, hematopoietic stem cells, and vascular endothelium progenitor cells.

In one embodiment of the method of the present invention, the step of selecting a cell population of the nucleated cells in which nuclei and SMN protein are labeled comprises measuring, in a single device, a first fluorescence intensity emitted by the first fluorescent dye and a second fluorescence intensity emitted by the second fluorescent dye. Moreover, in one embodiment of the method of the present invention, the step of selecting a cell population of the nucleated cells in which nuclei and SMN protein are labeled comprises measuring, in a single device, a first fluorescence intensity emitted by the first fluorescent dye and a second fluorescence intensity emitted by the second fluorescent dye, and representing the measured first and second fluorescence intensities in two-dimensional coordinates. In this case, it is sufficient to select a cell population in two-dimensional coordinates, and it is not always necessary to physically select a cell population. Sequentially measuring the first fluorescence intensity emitted by the first fluorescent dye and the second fluorescence intensity emitted by the second fluorescent dye in a single device makes it possible to ensure quick operation. In addition, selecting a cell population in two-dimensional coordinates also makes it possible to ensure quick operation.

As described above, the sample derived from blood obtained from a test subject, which is used in the method of the present invention, should contain nucleated cells derived from the blood. However, since whole blood (peripheral blood) contains all sorts of nucleated cells, it can be said that whole blood contains a cell population rich in diversity and thus heterogeneous. Further, since human blood cell fractions constantly vary, it is preferable to minimize the influence of the variation in blood cell fractions on the amount of the SMN proteins expressed in blood.

Hence, in the method of the present invention, nucleated cells in blood can also be classified into multiple clusters by using one or more surface antigen markers and the amount of the SMN protein expressed can be measured in each of the clusters. In this manner, the method of the present invention may comprise the steps of: classifying nucleated cells in blood into multiple clusters, for example, 2 to 4 clusters, by using one or more surface antigen markers; and selecting a predetermined cell population from the clusters. This is advantageous because it is possible to reduce the influence of the variation in blood cell fractions on the amount of the SMN proteins expressed in blood.

Note that although the method of the present invention comprises the step of selecting a cell population of the nucleated cells in which nuclei and SMN protein are labeled, the present invention may comprise the step of selecting a predetermined cell population from the clusters before, during, or after this selection step. For example, the method of the present invention may comprise the step of selecting a cell population of the nucleated cells in which nuclei and SMN protein are labeled, the cell population being selected from multiple clusters, for example, 2 to 4 clusters, classified from the nucleated cells by using one or more surface antigen markers.

The surface antigen markers which can be used in the method of the present invention include, but are not particularly limited to, CD45, CD66, CD14, CD3, CD19, CD33, CD123, CD34, CD11c, CD25, HLA-DR, and the like. In the method of the present invention, the surface antigen markers may be used alone, or two or more thereof may be used together. Among these surface antigen markers, CD45 is generally known as a panhemocyte marker; CD66 as a neutrophil marker; CD3 as a T cell marker; CD19 as a B cell marker; and CD14 as a monocyte marker.

Alternatively, in the method of the present invention, nucleated cells in blood may be classified into multiple clusters, for example, 2 to 4 clusters, based on side scatter (SSC) . In one embodiment of the method of the present invention, nucleated cells in blood can be classified into multiple clusters, for example, 2 to 4 clusters, by using one or more surface antigen markers and based on side scatter (SSC) . For example, the method of the present invention may comprise the step of selecting a cell population of the nucleated cells in which nuclei and SMN protein are labeled, the cell population being selected from multiple clusters, for example, 2 to 4 clusters, classified from the nucleated cells by using one or more surface antigen markers and based on side scatter (SSC).

One embodiment of the method of the present invention may comprise selecting a cell population of granulocytes or a cell population including granulocytes. One embodiment of the method of the present invention may comprise selecting a cell population of monocytes or a cell population including monocytes. One embodiment of the method of the present invention may comprise selecting a cell population of B cells or a cell population including B cells. One embodiment of the method of the present invention may comprise selecting a cell population of T cells or a cell population including T cells.

The method for detecting SMN protein expression of the present invention comprises a step of detecting SMN protein expression based on the label for the SMN protein in the cell population selected on the basis of the labels on the nuclei and the SMN protein in the nucleated cells, wherein said detection step comprises measuring an amount of the SMN protein expressed.

The method of the present invention makes it possible to enhance the sensitivity of detecting the level of the SMN protein expressed, by selecting a particular cell population and detecting the SMN protein expression in the cell population as described above. Moreover, in the method of the present invention, detection data based on the label for the SMN protein referred at the time of selecting the particular cell population can be used when the SMN protein expression is detected based on the label for the SMN protein in the selected cell population.

In one embodiment of the method of the present invention, the step of selecting a cell population of the nucleated cells in which nuclei and SMN protein are labeled with a fluorescent dye and the step of detecting SMN protein expression based on the label for the SMN protein in the selected cell population, wherein said detection step comprises measuring an amount of the SMN protein expressed, are performed by flow cytometry. Flow cytometry is suitable for quick treatments.

In one embodiment of the method of the present invention, the step of selecting a cell population of the nucleated cells in which nuclei and SMN protein are labeled and the step of detecting SMN protein expression based on the label for the SMN protein in the selected cell population are performed by imaging flow cytometry, wherein said detection step comprises measuring an amount of the SMN protein expressed. Since pictures of cells are taken at the same time in imaging flow cytometry, the fluorescence acquisition speed therein is set slower than those in general flow cytometry. Hence, more detailed analyses are possible.

Note that the flow cytometry and the imaging flow cytometry can be performed using commercially available devices according to manufacturer's instruction manuals thereof.

The method for detecting SMN protein expression of the present invention may comprise a step of comparing SMN protein expressions among subjects, for example, a childhood SMA patient, a carrier thereof, and a healthy person, specifically between a childhood SMA patient and a carrier thereof or between a childhood SMA patient and a healthy person. By the comparison, a difference in the amount of the SMN protein expressed is found between the childhood SMA patient and the carrier or between the childhood SMA patient and the healthy person, so that it is possible to classify the subject as a childhood SMA patient, a carrier, and a healthy person.

In addition, the method for detecting SMN protein expression of the present invention can also be used in a screening method for an agent for treating childhood SMA.

In the method of the present invention, it is possible to perform operations as appropriate, which are normally performed in detecting protein expression in cells, such as a step of washing cells with a reagent such as a phosphate buffer solution.

Additionally, in the method for detecting SMN protein expression of the present invention, the blood to be detected is obtained from a test subject, the step of detecting SMN protein expression comprises measuring an amount of the SMN proteins expressed, and the detection method may comprise a step of comparing the amount of the SMN protein expressed with a control. Here, the control may be selected from the following (a) to (c):
(a) if the test subject is a childhood SMA patient, the control is an amount of SMN protein expressed measured in the same manner as the process for measuring the expressed amount of the SMN protein by using the blood obtained from the test subject, except that blood obtained from a healthy person or a carrier is used;
(b) if the test subject is a healthy person or a carrier, the control is an amount of SMN proteins expressed measured in the same manner as the process for measuring the expressed amount of the SMN protein by using the blood obtained from the test subject, except that blood obtained from a childhood SMA patient is used; or
(c) if the test subject is a person to which a drug or a drug candidate against SMA has been administered, the control is an amount of SMN protein expressed measured in the same manner as the process for measuring the expressed amount of the SMN protein by using the blood obtained from the test subject after the administration of the drug or the drug candidate against SMA, except that blood obtained from the test subject before said administration is used.

Herein, the phrase "measured in the same manner as" should be construed to allow some modification that does not substantially influence the measurement of the amount of the SMN protein expressed in comparing the amounts of the SMN protein expressed. The phrase does not mean, for example, that the step of washing cells and so on are also completely the same.

The amount of the SMN protein expressed can be measured as appropriate by using techniques conventionally known in the technical field of the present invention, such as calibration curve method.

Note that one embodiment of the present invention comprises such a comparison step as described above, and also comprises a step of diagnosing the test subject based on the comparison result, and thus it can also be said that one embodiment of the present invention relates to a method for diagnosing childhood SMA. Moreover, one embodiment of the present invention comprises such a comparison step as described above, and also comprises a step of selecting a drug or a drug candidate against childhood SMA based on the comparison result, and thus it can also be said that one embodiment of the present invention relates to a screening method for a drug or a drug candidate against childhood SMA.

Heretofore, a quantitative analysis has been attempted on the SMN protein by the ELISA method using peripheral blood mononuclear cells (PBMCs) (Dione T. Kobayashi et al., Plos one, November 2012, Vol. 7, Issue 11, e50763, Evaluation of Peripheral Blood Mononuclear Cell Processing and Analysis for Survival Motor Neuron Protein). However, the ELISA method has disadvantages in that: cells are disrupted for the analysis; since the analyses have to be performed at the same time before and after the drug administration, the SMN protein may break down due to cell storage, freezing, or thawing treatments; and dead cells and extracellular SMN protein are also detected at the same time. In addition, although PBMCs are constituted of CD4+ and CD8+ T cells (approximately 65%), B cells and natural killer cells (approximately 28%), as well as CD14+ monocytes, granulocytes, and dendritic cells (approximately 7% in total), granulocytes, which are major constituents in blood, have a high relative density and precipitate to a lower layer during centrifugation. Hence, most of the granulocytes are not included in PBMCs which are located in an upper layer due to the relative density.

In contrast, according to the method for detecting SMN protein expression of the present invention, cells can be analyzed while keeping the shape, specimens can be treated on the same day (i.e., the freezing or thawing treatment is not necessary), and only the SMN protein in viable cells can be detected. Moreover, in the method for detecting SMN protein expression of the present invention, when target whole blood cells are classified into clusters by using surface antigen markers for the SMN protein analysis, the SMN protein analysis can be performed on a desired cell population (blood cell fractions), making it possible to reduce the influence of the variation in blood cell fractions on the amount of the SMN protein expressed.

### [Examples]

The present invention will be described in more details by way of Examples below. Note that the present invention is not limited to these Examples.

### Example 1: Detection of SMN Protein Expression Using Lymphoblasts

### (1) Lymphoblast Transformation Treatment

A blood sample obtained from a healthy person was collected into a heparin tube, then overlaid on a Ficoll solution, and centrifuged at 2000 rpm for 15 minutes. Thereby, peripheral blood mononuclear cells were separated and collected. Subsequently, a lymphoblast transformation treatment was performed by a transformation method using EB virus.

A blood sample obtained from a SMA type I patient was also subjected to the same treatment performed on the blood sample obtained from the healthy person.

### (2) Measurement of SMN Protein Expression

The lymphoblasts derived from the peripheral blood mononuclear cells of the healthy person obtained in (1) above were collected into a centrifuge tube. The cells were fixed with a 4% paraformaldehyde-phosphate buffer solution. Then, the cells were washed with a phosphate buffer solution and subsequently centrifuged at 500 x g for 5 minutes to remove the supernatant. As a cell membrane permeation reagent, BD Phosflow™ Perm Buffer II (BD Biosciences) was added to the resultant and left standing on ice for 30 minutes. And then, the cells were washed with BD pharmingen Stain Buffer (BD Biosciences), and a primary antibody: Purified Mouse Anti-SMN (Mouse monoclonal antibody, clone8/SMN, BD Biosciences) was then added for the reaction at room temperature for 60 minutes.

After that, the cells were washed with a phosphate buffer solution, and a secondary antibody (AlexaFluor 488 goat anti-mouse IgG (H+L), highly cross-absorbed, Life Technology) was added for the reaction at room temperature for 60 minutes while shielding light. The resultant was washed with a phosphate buffer solution. Then, Hoechst 33342 (Molecular Probes) was added to stain nuclei in the cells. Further, the cells were washed with a phosphate buffer solution. Subsequently, the SMN protein expression was measured by imaging flow cytometry (FlowSight(registered trademark), Amnis) .

The lymphoblasts derived from the peripheral blood mononuclear cells of the SMA type I patient obtained in (1) above were also measured for the SMN protein expression as in the case of the healthy person.

Note that, in the case of the healthy person, 10000 cells were used for the imaging flow cytometry, but cells which kept the cell form were focused on, so that 8970 cells were selected. Further, among these, 6302 cells which kept the aspect ratio (width-to-height ratio) and surface area were analyzed as shown in Fig. 1. Additionally, the same operations were performed again, so that a total of 20000 cells were used for the imaging flow cytometry.

In the case of the SMA type I patient also, 10000 cells were used for the imaging flow cytometry, but cells which kept the cell form were focused on, so that 8549 cells were selected. Further, among these, 4569 cells which kept the aspect ratio (width-to-height ratio) and surface area were analyzed as shown in Fig. 2. Additionally, the same operations were performed again, so that a total of 20000 cells were used for the imaging flow cytometry.

The analysis of SMN protein expression intensities were conducted by selecting cell populations 1 (populations 1) shown in Figs. 1 and 2. The cell populations shown in Figs. 1 and 2 were obtained by using 10000 cells from each of both the healthy person and the SMA type I patient. On the other hand, the graph in Fig. 3 shows distributions of fluorescence intensities of cell populations selected from 20000 cells from each of both the healthy person and the SMA type I patient.

Note that, in the cell populations 1, the nuclei and SMN protein were sufficiently fluorescence-labeled. Hence, the cell populations 1 were presumably intact cell groups. On the other hand, it cannot be said that the nuclei were sufficiently fluorescence-labeled in cell populations 2 (populations 2), and the cell populations 2 cannot be determined as intact cell groups, and were presumably a damaged cell group or a dead cell group. The numbers of cells in the cell populations 1 and 2 in Figs. 1 and 2 are as shown in Tables 1 and 2 below.

**Table 1**

| Fig. 1 (healthy persons) | Count (the number of cells) | Percentage (%) |
|---|---|---|
| Whole | 6302 | 100 |
| Cell population 1 (population 1) | 3174 | 50.4 |
| Cell population 2 (population 2) | 1596 | 25.3 |

**Table 2**

| Fig. 2 (SMA type I patient) | Count (the number of cells) | Percentage (%) |
|---|---|---|
| Whole | 4569 | 100 |
| Cell population 1 (population 1) | 1117 | 24.4 |
| Cell population 2 (population 2) | 832 | 18.2 |

Table 3 below shows the numbers of cells analyzed for the fluorescence intensities shown in Fig. 3 and the medians of the fluorescence intensities. Fig. 3 and Table 3 show the results obtained by using a total of 20000 cells for the imaging flow cytometry for each of both the healthy person and the SMA type I patient.

**Table 3**

| | Count (the number of cells) | Median |
|---|---|---|
| Normal control (whole) | 13417 | 66530.04 |
| Normal control (R2) | 12201 | 70913.87 |
| Normal control (R3) | 1215 | 21155 |
| SMA type I patient (whole) | 11451 | 10385 |
| SMA type I patient (R2) | 984 | 35985.43 |
| SMA type I patient (R3) | 10449 | 9562.4 |

From the above results, it was found that the normal control expressed the SMN protein approximately 6 times as much as the SMA type I patient by the comparison between the normal control (whole) and the SMA type I patient (whole) . Thus, the method of the present invention is capable of sensitive detection of SMN protein expression.

Note that R2 and R3 in Fig. 3 were to compare the fluorescence intensities by removing regions where the expression amounts overlapped between the normal control and the SMA type I patient for gating, and to show a difference between the two groups.

Meanwhile, frequency values on the vertical axis represent relative frequencies. The values used are relative values obtained according to proportions of cell populations having fluorescence intensities among the total cell populations.

### Example 2: Detection of SMN Protein Expression Using lysis Method

### (1) Lysis Treatment

2 mL of blood from a healthy person was collected into a heparin tube, which was inverted to mix well the blood. Then, 0.5 mL of the blood was collected into a centrifuge tube. BD Phosflow™ Lyse/Fix Buffer (BD Biosciences) was added thereto as a Lysing/Fix solution and left standing at 37°C for 10 minutes, followed by centrifugation at 2300 rpm for 8 minutes. After the supernatant was removed, the cells were washed with a phosphate buffer solution. A sample containing nucleated cells derived from the blood was thus obtained.

Samples containing nucleated cells derived from blood of a carrier and a SMA type I patient were also prepared by the lysis method as in the case of the healthy person.

### (2) Measurement of SMN Protein Expression

BD Phosflow™ Perm Buffer II (BD Biosciences) was added as a cell membrane permeation reagent to the nucleated cells of the healthy person obtained in (1) above, and left standing on ice for 30 minutes. And then, the cells were washed with BD pharmingen Stain Buffer (BD Biosciences), and Milli-Mark™ (anti-SMN antibody: Anti-SMN-FITC clone 2B1, Millipore) was then added for the reaction at room temperature for 60 minutes.

After that, the cells were washed with a phosphate buffer solution, and Hoechst 33342 (Molecular Probes) was added to stain nuclei in the cells. Further, the cells were washed with a phosphate buffer solution. Then, the SMN protein expression was measured by imaging flow cytometry (FlowSight(registered trademark), Amnis).

The samples of the carrier and the SMA type I patient obtained in (1) above were also measured for the SMN protein expression as in the case of the healthy person.

Moreover, the SMN protein expression intensities of intact cell groups in which nuclei and SMN protein were sufficiently fluorescence-labeled were analyzed in the same manner as in Example 1 above, except that 10000 cells from each of the healthy person, the carrier, and the SMA type I patient were used. Fig. 4 shows the results.

In addition, Table 4 below shows the numbers of cells analyzed for the fluorescence intensities shown in Fig. 4 and the medians of the fluorescence intensities.

**Table 4**

| | Count (the number of cells) | Median |
|---|---|---|
| Normal control | 7341 | 7936.19 |
| Carrier | 5616 | 3571.02 |
| SMA type I patient | 7810 | 1781.21 |

From the above results, differences in the SMN protein expression were clearly found among the healthy person, the carrier, and the SMA type I patient. Thus, the method of the present invention is capable of detecting a difference in the amount of the SMN protein expressed among a healthy person, a carrier, and a childhood SMA patient.

### Example 3: Detection of SMN Protein Expression in Each Cluster Classified Using Surface Antigen Markers

Samples were prepared according to the following staining protocol.
1. Collect 2 mL of peripheral blood obtained from a healthy person into a tube. Add reagents thereto such that 2 mL of the peripheral blood contains 80 µL of an Fc receptor blocking reagent (Clear Back, MTG-001, MBL) and 100 µL of Hoechst 33342 (Molecular Probes) which has been diluted to 5 µg/mL with PBS.
2. Seal the tube, and slowly rotate it at room temperature for 15 minutes in the dark.
3. Place 500 µL of the peripheral blood thus treated into each of four 15-mL conical tubes.
4. Add 5 µL of fluorochrome-labeled antibodies (purchased from BioLegend, Inc., BD Biosciences, and MERCK KGaA) into each of the tubes in accordance with Table 5 below.

**Table 5**

| | | Fluorochrome | | | | |
|---|---|---|---|---|---|---|
| | tube | PE | PE-Cy5 | BV510 | BV650 | BV785 |
| Monoclonal antibody | #1 | CD66a/c/e CD66b | CD33 | CD45 | CD11c | CD14 |
| | #2 | HLA-DR | CD123 | CD45 | CD11c | CD14 |
| | #3 | CD34 | CD123 | CD45 | CD19 | CD14 |
| | #4 | CD25 | CD3 | CD45 | CD19 | CD14 |

| | | | | | | |
|---|---|---|---|---|---|---|
| Monoclonal antibody clones: CD66a/c/e (ASL-32), CD66b (G10F5), CD34 (581), CD25 (M-A251), CD33 (WM53), CD123 (6H6), CD3 (UCHT1), CD45 (HI30), CD11c (3.9), CD19 (SJ25C1), CD14 (M5E2) (The data on CD123, CD34, CD11c, CD25, and HLA-DR are not shown in the present description.) PE: phycoerythrin PE-Cy5: phycoerythrin-cyanine 5 BV: brilliant violet | | | | | | |

5. Incubate the resultant at room temperature for 60 minutes in the dark.
6. Add 10 mL of a Lysing/Fix solution (BD Phosflow™ Lyse/Fix Buffer) which has been warmed with a water bath of 37°C into each tube (mix well the resultant by turning the tube upside down 8 to 10 times).
7. Incubate the resultant with a water bath of 37°C for 10 minutes.
8. Centrifuge the resultant at 500 x g at room temperature for 5 minutes.
9. Aspirate the supernatant using a disposable pipet.
10. Re-suspend the cells with 10 mL of PBS(-), and centrifuge the suspension in the same manner as in 8 above to remove the supernatant.
11. Re-suspend the cells with 1 mL of a cell membrane permeation reagent (BD Phosflow™ Perm/Wash Buffer I), and incubate the resultant at room temperature for 20 minutes.
12. Centrifuge the resultant in the same manner as in 8 above, and wash the cells twice with 2 mL of FBS/NaN₃ containing PBS (BD stain buffer 554656).
13. Place equal volumes of the cell suspension into two 1.5-mL microtubes, so that one tube contains approximately 5 x 10⁵ cells/45 µL.
14. Stain the cells with 5 µL of a FITC-conjugated anti-human SMN monoclonal antibody (2B1) or a FITC-conjugated anti-mouse IgG1 antibody (MOPC21; isotype control), and incubate the resultant on ice for 45 minutes in the dark.
15. Centrifuge the resultant in the same manner as in 8 above, followed by washing twice with 200 µL of FBS/NaN₃ containing PBS.
16. Re-suspend the cells with 50 µL of FBS/NaN₃ containing PBS.

The samples obtained as described above were measured for the SMN protein expression by imaging flow cytometry (FlowSight(registered trademark), Amnis).

First, cells in which the nuclei were stained and which kept the surface area, were selected in the same manner as shown in Fig. 5. Then, 73601 cells thus selected were classified into four clusters (Fig. 6; R1 to R4) by utilizing side scatter (SSC) and CD45. The number of cells in each of the clusters was as shown in Table 6 below.

**Table 6**

| | Count (the number of cells) | Percentage (%) |
|---|---|---|
| Whole | 73601 | 100 |
| R1 | 44677 | 60.7 |
| R2 | 12888 | 17.5 |
| R3 | 4088 | 5.55 |
| R4 | 12478 | 17 |

Further, after the adjustment was made to more clearly delimit the clusters, the fluorescence intensities emitted by the SMN protein labeling reagent were compared among the clusters (Fig. 7). Table 7 below shows the numbers of cells analyzed for the fluorescence intensities shown in Fig. 7 and the geometric means of the fluorescence intensities.

**Table 7**

| | Count (the number of cells) | Percentage (%) | Geometric mean |
|---|---|---|---|
| R1 | 30198 | 50.8 | 33543.08 |
| R2 | 8065 | 13.6 | 1600.38 |
| R3 | 2188 | 3.68 | 6951.74 |
| R4 | 9574 | 16.1 | 76692.48 |

Next, a cell population was further selected from the R1 cluster by utilizing CD33 and CD66abce (Fig. 8). As a result, it was revealed that 99.5% (the number of cells: 30060) of the R1 cluster in which SMN protein were labeled (the number of cells: 30198) were CD33+ CD66abce+ cells (neutrophil-like cells). The analysis was performed on fluorescence intensities emitted by the SMN protein labeling reagent in these CD33+ CD66abce+ cells (Fig. 9). The geometric mean of the fluorescence intensities emitted by the SMN protein labeling reagent was 33679.5.

Similarly, cell populations were further selected from the R2 cluster by utilizing CD3 and SMN protein or by utilizing CD19 and SMN protein, and the analysis was performed on fluorescence intensities emitted by the SMN protein labeling reagent (Figs. 10 and 11). As a result, it was revealed that 63.6% (the number of cells: 8197) of the R2 cluster in which SMN protein were labeled (the number of cells: 12882) were CD3+ cells (T cells), while 9.55% (the number of cells: 1230) thereof were CD19+ cells (B cells). Moreover, the geometric mean of the fluorescence intensities emitted by the SMN protein labeling reagent in the CD3+ cells shown in Fig. 10 was 1782.23, and the geometric mean of the fluorescence intensities emitted by the SMN protein labeling reagent in the CD19+ cells shown in Fig. 11 was 2103.25.

Next, a cell population was further selected from the R3 cluster by utilizing CD14 and SMN protein, and the analysis was performed on fluorescence intensities emitted by the SMN protein labeling reagent (Fig. 12). As a result, it was revealed that 61% (the number of cells: 2495) of the R3 cluster in which SMN protein were labeled (the number of cells: 4088) were CD14+ cells (monocyte-like cells). Moreover, the geometric mean of the fluorescence intensities emitted by the SMN protein labeling reagent in the CD14+ cells was 13606.79.

Summing up these results revealed that the expressed amount of the SMN protein, which is a ubiquitous protein, is increased in blood cells in the order of granulocytes (neutrophils, basophils, eosinophils) >monocytes > B cells and T cells. Accordingly, since the conventional SMN protein quantitative analysis by the ELISA method using PBMCs can hardly analyze granulocytes expressing a large amount of SMN protein, it is conceivable that the SMN protein expression cannot be analyzed sufficiently. On the other hand, the method of the present invention is capable of analyzing blood cells including granulocytes expressing a large amount of SMN protein, and also capable of analysis focusing on only granulocytes. These indicate that the method of the present invention is excellent in detecting SMN protein expression.

### Industrial Applicability

The method for completely curing SMA has not been established yet, and SMA is one of diseases designated as intractable by the country. The present invention is quite usefully utilized in evaluating a method which can be a SMA curing method. For example, the method of the present invention can be used in evaluating a drug or a drug candidate against SMA, and can be used in screening for a drug or a drug candidate against SMA. In addition, the present invention is usefully utilized in SMA diagnosis, too.

Therefore, remarkable technological advancements in diagnosing and treating SMA, which is one of intractable diseases, are expected from the present invention.

## Claims

1. A method for detecting the expression of survival motor neuron (SMN) protein, comprising the steps of:
labeling SMN protein in a sample containing nucleated cells derived from blood with a first fluorescent dye;
labeling the nuclei of the nucleated cells in the sample with a second fluorescent dye;
selecting a cell population of the nucleated cells whose nuclei and SMN protein are labeled; and
detecting the expression of the SMN protein based on the label for the SMN protein in the selected cell population, wherein said detection step comprises measuring an amount of the SMN protein expressed.

2. The method according to claim 1, wherein the step of detecting the expression of the SMN protein comprises measuring a fluorescence intensity emitted by the first fluorescent dye.

3. The method according to claim 1, wherein the step of selecting a cell population comprises:
measuring, in a single device, a first fluorescence intensity emitted by the first fluorescent dye and a second fluorescence intensity emitted by the second fluorescent dye, and
representing the measured first and second fluorescence intensities in two-dimensional coordinates.

4. The method according to any one of claims 1 to 3, wherein the step of selecting a cell population and the step of detecting the expression of the SMN protein are performed by imaging flow cytometry.

5. The method according to any one of claims 1 to 4, wherein the sample containing the nucleated cells derived from the blood includes nucleated cells obtained by hemolyzing red blood cells in the blood and then centrifuging the resultant to precipitate the nucleated cells.

6. The method according to any one of claims 1 to 5, comprising the step of centrifuging blood to prepare the sample containing nucleated cells derived from the blood before the step of labeling SMN protein in the sample containing the nucleated cells derived from the blood.

## Patentansprüche

1. Verfahren zur Detektieren der Expression von Survival-Motor-Neuron-(SMN-) Protein, umfassend die Schritte des:
Markierens von SMN-Protein in einer Probe, die nukleierte Zellen enthält, die von Blut abgeleitet sind, mit einem ersten fluoreszierenden Farbstoff,
Markierens der Nuklei der nukleierten Zellen in der Probe mit einem zweiten fluoreszierenden Farbstoff,
Wählens einer Zellpopulation der nukleierten Zellen, deren Nuklei und SMN-Protein markiert sind, und
Detektierens der Expression des SMN-Proteinsauf der Basis der Markierung für das SMN-Protein in der gewählten Zellpopulation, wobei der Detektionsschritt das Messen einer Menge des exprimierten SMN-Proteins umfasst.

2. Verfahren nach Anspruch 1, wobei der Schritt des Detektierens der Expression des SMN-Proteins das Messen einer Fluoreszenzintensität, die von dem ersten fluoreszierenden Farbstoff emittiert wird, umfasst.

3. Verfahren nach Anspruch 1, wobei der Schritt des Wählens einer Zellpopulation umfasst:
Messen, in einer einzigen Vorrichtung, einer ersten Fluoreszenzintensität, die von dem ersten fluoreszierenden Farbstoff emittiert wird, und einer zweiten Fluoreszenzintensität, die von dem zweiten fluoreszierenden Farbstoff emittiert wird, und
Darstellen der gemessenen ersten und zweiten Fluoreszenzintensitäten in zweidimensionalen Koordinaten.

4. Verfahren nach irgendeinem der Ansprüche 1 bis 3, wobei der Schritt des Wählens einer Zellpopulation und der Schritt des Detektierens der Expression des SMN-Proteins durch Bilddurchflusszytometrie ausgeführt werden.

5. Verfahren nach irgendeinem der Ansprüche 1 bis 4, wobei die Probe, die die nukleierten Zellen enthält, die von dem Blut abgeleitet sind, nukleierte Zellen enthält, die durch Hämolysieren roter Blutzellen in dem Blut und daraufhin Zentrifugieren des Resultierenden, um die nukleierten Zellen auszufällen, erhalten werden.

6. Verfahren nach einem der Ansprüche 1 bis 5, umfassend den Schritt des Zentrifugierens von Blut, um die Probe, die die nukleierten Zellen, die von dem Blut abgeleitet sind, enthält, vor dem Schritt des Markierens des SMN-Proteins in der Probe, die die nukleierten Zellen enthält, die von dem Blut abgeleitet sind, herzustellen.

## Revendications

1. Procédé pour détecter l'expression de la protéine de survie des motoneurones (SMN), comprenant les étapes de:
marquer la protéine SMN dans un échantillon contenant des cellules nucléées dérivées du sang avec un premier colorant fluorescent;
marquer les noyaux des cellules nucléées dans l'échantillon avec un second colorant fluorescent;
sélectionner une population cellulaire des cellules nucléées dont les noyaux et la protéine SMN sont marqués; et
détecter l'expression de la protéine SMN sur la base du marqueur pour la protéine SMN dans la population cellulaire sélectionnée, dans lequel ladite étape de détection comprend la mesure d'une quantité de la protéine SMN exprimée.

2. Procédé selon la revendication 1, dans lequel l'étape de détection de l'expression de la protéine SMN comprend la mesure d'une intensité de fluorescence émise par le premier colorant fluorescent.

3. Procédé selon la revendication 1, dans lequel l'étape de sélection d'une population cellulaire comprend:
la mesure, dans un seul dispositif, d'une première intensité de fluorescence émise par le premier colorant fluorescent et d'une seconde intensité de fluorescence émise par le second colorant fluorescent, et
la représentation des première et seconde intensités de fluorescence mesurées en coordonnées en deux dimensions.

4. Procédé selon l'une quelconque des revendications 1 à 3, dans lequel l'étape de sélection d'une population cellulaire et l'étape de détection de l'expression de la protéine SMN sont accomplies par cytométrie en flux d'imagerie.

5. Procédé selon l'une quelconque des revendications 1 à 4, dans lequel l'échantillon contenant les cellules nucléées dérivées du sang inclut des cellules nucléées obtenues par hémolyse des globules rouges dans le sang puis centrifugation de ce qui en résulte pour précipiter les cellules nucléées.

6. Procédé selon l'une quelconque des revendications 1 à 5, comprenant l'étape de centrifugation du sang pour préparer l'échantillon contenant des cellules nucléées dérivées du sang avant l'étape de marquage de la protéine SMN dans l'échantillon contenant les cellules nucléées dérivées du sang.
